# EUROPÄISCHE PATENTSCHRIFT

(11) **EP 3 217 981 B1**
(45) Veröffentlichungstag und Bekanntmachung des Hinweises auf die Patenterteilung: **31.07.2019**
(21) Anmeldenummer: 15795132.8
(22) Anmeldetag: 04.11.2015
(51) Int. Cl.: A61K 31/455, A61K 35/644, A61K 36/31, A61K 8/67, A61Q 19/00, A61K 8/97, A61K 8/98, A61K 31/07, A61K 31/164, A61K 31/375, A61K 31/496, A61K 31/60, A61P 17/10

(54) **KOSMETISCHE ZUSAMMENSETZUNG UND DEREN VERWENDUNG ZUR PROPHYLAXE UND BEHANDLUNG VON HAUTUNREINHEITEN, ZU AKNE NEIGENDER HAUT UND AKNE**
COSMETIC COMPOSITION, ITS PROPHYLACTIC USE AND IN TREATMENT OF SKIN IMPURITIES, OF SKIN SUSCEPTIBLE TO ACNE AND ACNE
COMPOSITION COSMÉTIQUE, SON UTILISATION PROPHYLACTIQUE ET POUR LE TRAITEMENT DES IMPURETÉS DE LA PEAU, DE LA PEAU AYANT UNE TENDANCE À L'ACNÉ ET DE L'ACNÉ

(30) Priorität: 14.11.2014 DE 102014116691
(43) Veröffentlichungstag der Anmeldung: 20.09.2017
(73) Patentinhaber: Cosmedes GmbH, 64625 Bensheim (DE)
(72) Erfinder: HAMBEK, Daniela, 64625 Bensheim (DE)
(74) Vertreter: Habermann Intellectual Property Partnerschaft von Patentanwälten mbB
(86) Internationale Anmeldenummer: PCT/EP2015/075760
(87) Internationale Veröffentlichungsnummer: WO 2016/075019

(56) Entgegenhaltungen:
- EP-A1- 2 711 050
- US-A1- 2010 129 305
- US-A1- 2011 020 302
- DATABASE GNPD [Online] MINTEL; September 2012 (2012-09), "Oil Free Matte SPF 30", XP002752833, Database accession no. 1869386
- DATABASE GNPD [Online] MINTEL; September 2014 (2014-09), "Spot Correction Acne Eliminating Treatment", XP002752834, Database accession no. 2698595
- DATABASE GNPD [Online] MINTEL; Juli 2013 (2013-07), "Propolis Face Gel", XP002752835, Database accession no. 2102515
- DATABASE GNPD [Online] MINTEL; Mai 2011 (2011-05), "Anti-Acne Gel", XP002752836, Database accession no. 1542732

## Beschreibung

Die vorliegende Erfindung betrifft eine Leuconostoc Radieschenwurzel Ferment Filtrat aufweisende kosmetische Zusammensetzung und deren Verwendung zur Prophylaxe und Behandlung von Hautunreinheiten, zu Akne neigender Haut und Akne.

Unreine, zu Akne neigende Haut und Akne sind Hautprobleme, die abhängig vom Lebensalter der betroffenen Person eine unterschiedliche Behandlung benötigen. Auf dem Markt sind zahlreiche Produkte erhältlich, die oftmals stark austrocknend oder komedogen wirken. Insbesondere mit zunehmendem Lebensalter lässt die Regenerationsfähigkeit der Haut nach und die Haut reagiert irritiert auf aggressive Pflegeprodukte. Ferner berichten Personen im mittleren Lebensalter mit diesem Hautbild oftmals, dass sie auf praktisch jede angebotene Pflege mit einer Verschlechterung der Hautsituation reagieren und daher mitunter auf eine Pflege ganz verzichten.

Leuconostoc Radieschenwurzel Ferment Filtrat ist ein kosmetischer Wirkstoff, der nach der Gärung der Wurzeln der Gattung Raphanus sativus Wurzeln mit dem Mikroorganismus Leuconostoc als Filtrat erhältlich ist. Das Filtrat wird aufgrund seiner antimikrobiellen Wirkung bereits als Antischuppenwirkstoff genutzt. Aus der japanischen Patentanmeldung JP 2009 191019 A ist ein Leuconostoc Radieschenwurzel Ferment Filtrat enthaltendes kosmetisches Produkt bekannt, das unter anderem zur Behandlung und Vorbeugung von Akne, rauer und alternder Haut eingesetzt werden kann.

Vor diesem Hintergrund liegt der vorliegenden Erfindung die Aufgabe zugrunde, eine Leuconostoc Radieschenwurzel Ferment Filtrat enthaltende kosmetische Zusammensetzung zu schaffen, die eine verbesserte Wirksamkeit für die Prophylaxe und Behandlung von Hautunreinheiten und Akne aufweist, wobei insbesondere die spezielle Hautphysiologie der Problemhaut von Personen im mittleren Lebensalter zu berücksichtigen ist.

Diese Aufgabe wird durch eine kosmetische Zusammensetzung gelöst, die die ein Wirkstoffgemisch aus Leuconostoc Radieschenwurzel Ferment Filtrat, Propolis und Nicotinamid aufweist.

Es hat sich überraschend herausgestellt, dass die Wirkstoffkomponenten Leuconostoc Radieschenwurzel Ferment Filtrat, Propolis und Nicotinamid nach Auftragen auf von Akne betroffene Hautstellen ein schnelles Abheilen der Pickel und Komedonen und eine vollständige Heilung bewirken, ohne dass auch bei reifer und empfindlicher Haut Hautirritationen festgestellt werden konnten. Diese Wirkung ist bereits bei geringen Anteilen des Wirkstoffgemisches in der Zusammensetzung deutlich feststellbar. Der Anteil der Wirkstoffkomponenten in den erfindungsgemäßen Zusammensetzungen beträgt demnach abhängig von der Art der Formulierung vorzugsweise 0,01 bis 2 Gew.-% Leuconostoc Radieschenwurzel Ferment Filtrat, 0,01 bis 2 Gew.-% Propolis und 0,1 bis 2 Gew.-% Nicotinamid bezogen auf die jeweilige Zusammensetzung.

Im Sinne dieser Erfindung ist mit Propolis das von Bienen hergestellte Bienenharz gemeint, das als Feststoff, als Tinktur oder als Extrakt in einer erfindungsgemäßen Zusammensetzung vorliegen kann.

Die erfindungsgemäßen Zusammensetzungen können in einer beliebigen zum Auftragen auf die Haut geeigneten Form vorliegen, z.B. in Form eines Gels, einer Lotion, einer Creme, eines Schaums, eines Serums, eines Öls, einer Salbe, einer Paste, einer Emulsion, einer Suspension, einer Lösung, eines Puders, einer Tinktur, eines Make-ups und dergleichen. Die Auswahl eines für die gewünschte Form geeigneten Trägers, Grundstoffs oder Verdünnungsmittels liegt im Bereich des Könnens von Fachleuten auf dem Gebiet.

In einer vorteilhaften Ausgestaltung der Erfindung weist die erfindungsgemäße kosmetische Zusammensetzung weiterhin einen entzündungshemmenden Wirkstoff auf. Der entzündungshemmende Wirkstoff trägt zu einer Beschleunigung des Heilungsprozesses der von Akne betroffenen Hautpartien bei.

Bevorzugte entzündungshemmende Wirkstoffe sind Gelee Royal, Salicylsäure, Panthenol, Retinol, Elubiol, Ascorbinsäure, ein Pflanzenextrakt und Mischungen daraus, wobei Gelee Royal besonders bevorzugt ist.

Ein besonders geeigneter Pflanzenextrakt ist Hamamelis-Extrakt, der eine adstringierende, blutstillende, entzündungshemmende und Juckreiz stillende Wirkung aufweist und sehr gut hautverträglich ist.

Weitere geeignete entzündungshemmende Pflanzenextrakte können aus Borretsch (Borago officinalis), Nachtkerze (Oenothera biennis), Sonnenhutkraut (Echinacea angustifolia), Ginkgo (Ginkgo biloba), Kamille (Chamomilla recucita, C. nobile, Matricaria matricarioides) Arnika (Arnica chamissonis, A. montana), Ringelblume (Calendula officinalis), Thymian (Thymus spp.), Salbei (Salvia officinalis), Minze (Mentha spp.), Johanniskraut (Hypericum perforatum), Grapefruit (Citrus aurantium, C. paradisi), Alge (Laminaria saccharina oder digitata), Chinarindenbaum (Cinchona spp.), Rosmarin (Rosmarinus officinalis), Acerola (Malpighia glabra, Syn.: Malpighia punicifolia), Oregano (Origanum vulgare), Sanddorn (Hippophae rhamnoides), Kürbiskernextrakt, Cardiospermum halicacabum, Myrrhe (Commiphora spp.), Ratanhia, Fenchel (Foeniculum vulgare), Weide (Salix spp.), Schafgarbe (Achillea millefolium), Hibiscus, Huflattich (Tussilago farfara), Beinwell (Symphytum officinale), Grünteeextrakt, Weissteeextrakt, Teufelskralle (Hapagophytum procumbens), Bittersüß (Solanum dulcamara), Holunder (Sambus nigra), Eucalyptus, Teebaum (Melaleuca alternifolia), Teestrauch (Camillia sinensis), Süßholz (Glycyrrhiza spp.), Melisse (Melissa officinalis), Koriander, Campher (Cinnamomum camphora), Eucalyptus spp., Tausendgüldenkraut (Centaurium erythraea), Brennessel (Urtica spp.), Quitte (Cydonia oblonga), Ananas, Fichte, Kiefer oder Eiche gewonnen werden.

Die erfindungsgemäß geeigneten entzündungshemmenden Pflanzenextrakte können beispielsweise Gerbstoffe, ätherische Öle, Azulene, Proazulene, Bisabolole, Bisaboloide, Flavonoide, Flavone, Anthocyane, Triterpene, Monoterpenalkohole, Phenolcarbonsäuren, Polyphenole, ungesättigte Fettsäuren, Hypericin, Carotinoide, Allantoin, Bromelain, Glycyrrhizin, Glycyrrhizinsäure oder pharmazeutisch akzeptable Salze der Glycorrhizinsäure sowie Mischungen daraus sein. Weiterhin kommen Nachtkerzenöl, Borretschöl sowie Weizenkeimöl in Betracht.

Pharmazeutisch akzeptable Salze können Säureadditionssalze mit Mineralsäuren, Carbonsäuren oder Sulfonsäuren sein. Besonders bevorzugt sind z.B. Salze mit Schwefelsäure, Essigsäure, Propionsäure, Milchsäure, Weinsäure, Zitronensäure, Fumarsäure, Alaune, Maleinsäure, Oxalsäure, Asparaginsäure oder Benzoesäure.

Zur Gewinnung der Pflanzenextrakte werden insbesondere die Blätter, Wurzeln, Blüten, Früchte oder Rinde der Pflanzen verwendet. Der Pflanzenextrakt kann ein Einzelstoff, ein Stoffgemisch, ein flüssiger oder fester bzw. trockener Extrakt, ein Destillat oder ein Öl sein. Die jeweils geeigneten Pflanzenteile und Extraktionsverfahren sind Fachleuten auf dem Gebiet bekannt.

Der Anteil der entzündungshemmenden Wirkstoffe in der kosmetischen Zusammensetzung beträgt vorzugsweise 0,05 bis 75 Gew.-%, besonders bevorzugt 0,1 bis 50 Gew.-% bezogen auf die jeweilige Zusammensetzung.

In einer weiteren vorteilhaften Ausgestaltung der Erfindung weist die erfindungsgemäße Zusammensetzung weiterhin einen feuchtigkeitsspendenden Wirkstoff auf. Besonders bevorzugt ist der feuchtigkeitsspendende Wirkstoff ausgewählt aus Milchsäure, Hyaluronsäure und deren pharmazeutische zulässigen Salze, Aloe Vera, Harnstoff oder einer Mischung daraus.

Der Anteil der feuchtigkeitsspendenden Wirkstoffe in der kosmetischen Zusammensetzung beträgt vorzugsweise 0,01 bis 20 Gew.-%, besonders bevorzugt 0,1 bis 10 Gew.-% bezogen auf die jeweilige Zusammensetzung.

In noch einer weiteren vorteilhaften Ausgestaltung der Erfindung weist das Wirkstoffgemisch einen weiteren aktiven Wirkstoff auf. Der weitere aktive Wirkstoff kann insbesondere pflegende und glättende Eigenschaften für die Haut aufweisen, die für bestimmte Hauttypen wünschenswert und von Vorteil sind. Bevorzugte aktive Wirkstoffe sind beispielsweise Squalan, Aprikosenkernöl, Traubenkernöl, Hagebuttenkernöl, Allantoin, Gelee Royal, Seidenpulver, Vitamin A, Vitamin C, Vitamin E und Mischungen daraus.

Der Anteil der weiteren aktiven Wirkstoffe in der kosmetischen Zusammensetzung beträgt vorzugsweise 0,01 bis 20 Gew.-%, besonders bevorzugt 0,1 bis 10 Gew.-% bezogen auf die jeweilige Zusammensetzung.

Zur Vermeidung möglicher Hautirritationen oder Allergien auslösender Reaktionen ist es wünschenswert, dass die erfindungsgemäßen Zusammensetzungen möglichst wenige weitere Zusatzstoffe enthalten. Jedoch kann es beispielsweise zur Erlangung einer verbesserten Löslichkeit, einer verlängerten Haltbarkeit oder einer erhöhten Verbraucherakzeptanz vorteilhaft sein, der erfindungsgemäßen Zusammensetzung Zusatz- und Hilfsstoffe, wie z.B. Emulgatoren, Feuchthaltemittel, Konservierungsstoffe, Antioxidantien, Stabilisatoren, peelende Stoffe, UV-Absorber, Farbstoffe, Duftstoffe und dergleichen zuzugeben.

Geeignete Emulgatoren sind beispielsweise Wollwachs, Sorbitanester, Monoglyceride, Glycerin, Glykolderivate und Mischungen daraus.

Als Feuchthaltemittel können beispielsweise Sorbitol, Glycerin, Propylenglykol, Butylenglykol, Hexylenglykol, Xylitol, Lactitol, Fructose, Glucose, Mannose, Xylose, Honig oder Mischungen daraus eingesetzt werden.

Besonders bevorzugte Konservierungsstoffe sind z.B. Benzalkoniumchlorid und Parabene.

Zur Verbesserung der Reinigungswirkung können in den erfindungsgemäßen Zusammensetzungen außerdem peelende, d.h. abschuppende Stoffe enthalten sein. Geeignete peelende Stoffe sind beispielsweise Glykolsäure, Citronensäure, Mandelsäure, Äpfelsäure, Retinol und Benzoylperoxid.

Die erfindungsgemäßen Zusammensetzungen können weiterhin UV-Absorber aufweisen, wie z.B. Benzophenon-3, Benzophenon-4, Benzophenon-5, 3-Benzylidencampher, Benzylidencampher Sulfonsäure, Bis-Ethylhexyloxyphenol-methoxyphenyltriazin (BEMT), Butylmethoxydibenzoylmethan (BMDM, Avobenzon), Diethylhexylbutamidotriazon, Dimethicodiethylbenzalmalonat, Dimethyl Pabamidopropyllaurdimonium-tosylat, Drometrizoltrisiloxan (DTS), Ethylhexyldimethyl PABA, Ethylhexylmethoxycinnamat, Ethylhexylsalicylat (EHS), Homosalate, Isoamyl-p-methoxycinnamat, Isopropylbenzylsalicylat, 4-Methylbenzylidencampher, Methylen-bis-benzotriazolyl-tetramethylbutylphenol (MBBT), Octocrylen (OC), Octyltriazon, PABA, PEG-25 PABA, Phenylbenzimidazol Sulfonsäure (PBSA), Dinatrium-Phenyldibenzimidazoltetrasulfonat (DPDT), Polyacrylamidomethylbenzylidencampher, Terephthalidendicampher Sulfonsäure (TDSA) und deren pharmazeutische zulässigen Salze, Titandioxid, Zinkoxid oder Magnesiumoxid.

Die erfindungsgemäßen Zusammensetzungen können noch weitere Stoffe, beispielsweise mattierende Stoffe, wie z.B. Kaolin, Reisstärke, Maisstärke, Talk, Kürbissamenextrakt, Cellulosemicrobeads, Pflanzenfasern, synthetische Fasern, Silizium, und Titandioxid, adstringierende Stoffe, wie z.B. Alcloxa, hautaufhellende Stoffe, wie z.B. Resorcinol, Duftstoffe, wie z.B. etherische Öle, und dergleichen enthalten.

Typische erfindungsgemäße Zusammensetzungen für eine Hautcreme können folgende Bestandteile enthalten:

**Formulierung I**

| | |
|---|---|
| Wasser | >50% |
| Aprikosenkernöl | 1%-10% |
| Squalan | 1%-5% |
| Panthenol | 1%-5% |
| Methylpropandiol | 1%-5% |
| Sorbitanstearat | 1%-5% |
| Methylglucose Sesquistearat | 1%-5% |
| Harnstoff oder Glycerin | 1%-5% |
| Nicotinamid | 0,1%-2% |
| Hamameliswasser | 1%-5% |
| Alkohol (denaturiert) | 0,1%-10% |
| Zitronensäure | 0,1%-1% |
| Caprylylglycol | 0,1%-2% |
| Phenylpropanol | 0,1%-1% |
| Natriumhyaluronat | 0,1%-2% |
| Propolisextrakt | 0,01%-2% |
| Milchsäure | 0,01%-0,5% |
| Leuconostoc/Radieschenwurzel Ferment Filtrat | 0,01%-2% |

**Formulierung II**

| | |
|---|---|
| Wasser | >50% |
| Capryl-/Caprin-triglycerid (Neutralöl) | 25%-50% |
| Squalan | 0,1%-5% |
| Harnstoff oder Glycerin | bis zu 5% |
| Methylpropandiol | 1%-5% |
| Sorbitanstearate | 1%-5% |
| Methylglucose Sesquistearat | 1%-5% |
| Traubenkernöl | 0,1%-5% |
| Aprikosenkernöl | 0,1%-5% |
| Nicotinamid | 0,1%-2% |
| Allantoin | 0,1%-1% |
| Propolisextrakt | 0,1%-2% |
| Caprylylglycol | 0,1%-1% |
| Phenylpropanol | 0,1%-1% |
| Natriumhyaluronat | 0,1%-2% |
| Aloe Barbadensis Blatt Extrakt | 0,1%-1% |
| Leuconostoc/Radieschenwurzel Ferment Filtrat | 0,01% - 2% |
| Milchsäure | <0,1% |
| Gelee Royal | 0,1%-1,0% |
| Hydrolysierte Seide | <1% |
| Zitronensäure | <0,1% |
| Natriumbenzoat | <0,1% |
| Kaliumsorbat | <0,1% |

Die Erfindung ist anhand von Ausführungsbeispielen nachstehend weiter erläutert.

### Formulierungsbeispiel 1: Herstellung einer Creme

Für die Herstellung einer ersten Phase werden 45 g Emulgator (Gemisch aus Sorbitan Stearat und Methyl Glucose Sesquistearat), 20 g Squalan und 50 g Aprikosenkernöl nacheinander in ein hitzefestes Gefäß einwogen, auf 70 °C erhitzt und mit einem Spatel bis zur Schmelze gerührt. Für die Herstellung einer zweiten Phase werden 643,5 g Wasser in einem hitzebeständigen Gefäß aufkochen und danach auf 70 °C abkühlen gelassen. Anschließend werden zu dem Wasser 10 g Nicotinamid, 20 g Dexpanthenol, 40 g einer Additivzusammensetzung aus Methylpropandiol, Caprylyl Glycol und Phenylpropanol, 20 g Hamamelisrindenwasser, 20 g einer Mischung aus Propolisextrakt und Leuconostoc/Radieschenwurzel Ferment Filtrat sowie 0,5 g Milchsäure gegeben und mit einem Spatel verrührt. Unter Verwendung eines elektrischen Rührers, mit dem wenig Luft eingearbeitet wird, wird 1 g Natriumhyaluronat eingestreut. Diese zweite Phase wird ca. 1 Stunde quellen gelassen.

Die zweite Phase wird unter Verwendung eines elektrischen Rührgeräts langsam in die erste Phase eingerührt, bis eine Emulsion erhalten wird. Der pH-Wert der erhaltenen Emulsion wird geprüft und gegebenenfalls mit Milchsäure auf unter pH 6 eingestellt.

Für die Herstellung einer dritten Phase werden 100 g Wasser in einem hitzebeständigen Gefäß aufkochen und danach auf Raumtemperatur abkühlen gelassen. Der pH-Wert wird mit Milchsäure auf pH < 6 eingestellt. 30 g Harnstoff werden bis zur vollständigen Lösung eingerührt. Die so erhaltene dritte Phase wird in die aus der ersten und der zweiten Phase gebildete Emulsion eingerührt und bis zum Erreichen der Raumtemperatur regelmäßig aufgerührt. Der pH-Wert der erhaltenen Creme wird überprüft und gegebenenfalls mit Milchsäure auf ca. pH 5,5 eingestellt.

### Formulierungsbeispiel 2: Herstellung einer Creme

Für die Herstellung einer ersten Phase werden 50 g Emulgator (Gemisch aus Sorbitan Stearat und Methyl Glucose Sesquistearat), 20 g Squalan, 10 g Traubenkernöl, 10 g Aprikosenkernöl und 300 g Neutralöl nacheinander in ein hitzefestes Gefäß einwogen, auf 70 °C erhitzt und bis zur Schmelze gerührt. Für die Herstellung einer zweiten Phase werden 520,5 g Wasser in einem hitzebeständigen Gefäß aufkochen und danach auf 70 °C abkühlen gelassen. Anschließend werden zu dem Wasser 5 g Allantoin, 10 g Nicotinamid, 1 g Gelee Royal, 40 g einer Additivzusammensetzung aus Methylpropanediol, Caprylyl Glycol und Phenylpropanol, 2 g Aloe Vera Konzentrat (10:1), 10 g Seidenextrakt, 20 g einer Mischung aus Propolisextrakt und Leuconostoc/Radieschenwurzel Ferment Filtrat sowie 0,5 g Milchsäure gegeben und verrührt. Zur Durchführung des Rührvorgangs wird ein elektrischer Rührer verwendet, mit dem wenig Luft eingearbeitet wird. Während des Rührvorgangs wird 1 g Natriumhyaluronat eingestreut. Diese Phase wird ca. 1 Stunde quellen gelassen.

Die zweite Phase wird langsam in die erste Phase eingerührt bis eine bei Zimmertemperatur stabile Emulsion erhalten wird. Der pH-Wert der erhaltenen Creme wird geprüft und gegebenenfalls mit Milchsäure auf ca. pH 5,5 eingestellt.

### Beispiel 3:

Eine 42jährige körperlich gesunde Patientin litt seit der Pubertät an unreiner Haut und zahlreichen Pickeln. Vor Anwendungsbeginn zählte man im Gesicht 26 Pickel und 139 Komedonen. Nach acht Wochen zweimal täglicher Anwendung einer Creme gemäß Beispiel 2 reduzierten sich die Anzahl der Pickel auf 7 und die Anzahl der Komedonen auf 23. Die Zufriedenheit der Anwenderin verbesserte sich (im Schulnotensystem von 1 bis 6) von 5 auf 2.

### Beispiel 4:

Eine 29jährige Patientin mit einer Autoimmunerkrankung beklagte seit vier Jahren Pickel und Komedonen. Vor Anwendungsbeginn zählte man 39 Pickel und 109 Komedonen im Gesicht. Die Patientin behandelte die Gesichtshaut zweimal täglich mit einer Creme gemäß Beispiel 1. Bereits nach vier Wochen reduzierten sich die Anzahl der Pickel auf 14 und die Anzahl der Komedonen auf 47. Die subjektive Zufriedenheit der Patientin verbesserte sich (im Schulnotensystem von 1 bis 6) von 5 auf 2.

## Patentansprüche

1. Kosmetische Zusammensetzung, die ein Wirkstoffgemisch aus Leuconostoc Radieschenwurzel Ferment Filtrat, Propolis und Nicotinamid aufweist.

2. Kosmetische Zusammensetzung nach Anspruch 1, die weiterhin einen entzündungshemmenden Wirkstoff aufweist.

3. Kosmetische Zusammensetzung nach Anspruch 1 oder 2, bei der der entzündungshemmende Wirkstoff ausgewählt ist aus Gelee Royal, Salicylsäure, Panthenol, Retinol, Elubiol, Ascorbinsäure, einem Pflanzenextrakt oder einer Mischung daraus.

4. Kosmetische Zusammensetzung nach einem der vorangehenden Ansprüche, die weiterhin einen feuchtigkeitsspendenden Wirkstoff aufweist.

5. Kosmetische Zusammensetzung nach Anspruch 4, bei der der feuchtigkeitsspendende Wirkstoff ausgewählt ist aus Milchsäure, Hyaluronsäure und deren pharmazeutische zulässigen Salze, Aloe Vera, Harnstoff oder einer Mischung daraus.

6. Kosmetische Zusammensetzung nach einem der vorangehenden Ansprüche, die einen weiteren aktiven Wirkstoff aufweist.

7. Kosmetische Zusammensetzung nach Anspruch 6, bei der der aktive Wirkstoff ausgewählt ist aus Squalan, Aprikosenkernöl, Traubenkernöl, Hagebuttenkernöl, Allantoin, Seidenpulver, Vitamin A, Vitamin C, Vitamin E oder einer Mischung daraus.

8. Nicht-therapeutische Verwendung einer kosmetischen Zusammensetzung nach einem der vorangehenden Ansprüche zur Behandlung von Hautunreinheiten.

9. Kosmetische Zusammensetzung nach einem der Ansprüche 1 bis 7 zur Anwendung in der Prophylaxe oder Behandlung von zu Akne neigender Haut und Akne.

## Claims

1. Cosmetic composition, which comprises an active substance mixture of Leuconostoc radish root ferment filtrate, propolis and nicotinamide.

2. Cosmetic composition according to claim 1, which further comprises an anti-inflammatory substance.

3. Cosmetic composition according to claim 1 or 2, in which the anti-inflammatory substance is selected from royal jelly, salicylic acid, panthenol, retinol, elubiol, ascorbic acid, a plant extract, or a mixture thereof.

4. Cosmetic composition according to any one of the preceding claims, which further comprises a moisturizing active substance.

5. Cosmetic composition according to claim 4, in which the moisturizing active substance is selected from lactic acid, hyaluronic acid, and their pharmaceutically permissible salts, aloe vera, urea, or a mixture thereof.

6. Cosmetic composition according to any one of the preceding claims, which comprises a further active substance.

7. Cosmetic composition according to claim 6, in which the active substance is selected from Squalane, apricot kernel oil, grape seed oil, rosehip seed oil, allantoin, silk powder, vitamin A, vitamin C, vitamin E, or a mixture thereof.

8. Non-therapeutic use of a cosmetic composition according to any one of the preceding claims, for the treatment of skin impurities.

9. Cosmetic composition according to any one of claims 1 to 7, for use in the prophylaxis or treatment of skin susceptible to acne and acne.

## Revendications

1. Composition cosmétique, qui présente un mélange de principes actifs composé de leuconostoques, de racine de radis, d'un ferment, d'un filtrat, de propolis et de nicotinamide.

2. Composition cosmétique selon la revendication 1, qui présente par ailleurs un principe actif anti-inflammatoire.

3. Composition cosmétique selon la revendication 1 ou 2, où le principe actif anti-inflammatoire est choisi parmi la gelée royale, l'acide salicylique, le panthénol, le rétinol, l'élubiol, l'acide ascorbique, un extrait de plante ou un mélange de ces derniers.

4. Composition cosmétique selon l'une quelconque des revendications précédentes, qui présente par ailleurs un principe actif hydratant.

5. Composition cosmétique selon la revendication 4, où le principe actif hydratant est choisi parmi l'acide lactique, l'acide hyaluronique et ses sels pharmaceutiquement acceptables, l'aloe vera, l'urée ou un mélange de ces derniers.

6. Composition cosmétique selon l'une quelconque des revendications précédentes, qui présente un autre principe actif.

7. Composition cosmétique selon la revendication 6, où le principe actif est choisi parmi le squalane, l'huile de noyau d'abricot, l'huile de pépins de raison, l'huile de graines de cynorrhodon, l'allantoïne, la poudre de soie, la vitamine A, la vitamine C, la vitamine E ou un mélange de ces derniers.

8. Utilisation non thérapeutique d'une composition cosmétique selon l'une quelconque des revendications précédentes servant au traitement des impuretés de la peau.

9. Composition cosmétique selon l'une quelconque des revendications 1 à 7 destinée à être utilisée dans la prophylaxie ou le traitement d'une peau à tendance acnéique et de l'acné.
